# EUROPEAN PATENT APPLICATION

(11) **EP 3 181 176 A1**
(43) Date of publication of application: **21.06.2017**
(21) Application number: 15199934.9
(22) Date of filing: 14.12.2015
(51) Int. Cl.: A61M 25/01, A61B 17/00

(54) **MAGNETICALLY GUIDED MEDICAL DEVICE**

(71) Applicant: Aeon Scientific AG, 8952 Schlieren (CH)
(72) Inventor: LYTTLE, Sean, 8052 Zürich (CH)
(74) Representative: Liebetanz, Michael

(57) **Abstract**

A device (10) for navigation within a patient's body, said apparatus comprising a tubular structure having an inner lumen and relatively rigid proximal (15) and distal portions (14) separated by at least one very flexible joint (12) with constant inner diameter and small bending radius. At least one magnetic element (13) is embedded in the distal portion of the device and responsive to externally applied magnetic fields, such that the orientation of distal portion aligns approximately with an externally applied magnetic field (B). The inner lumen of the device allows a flexible medical instrument (17) (e.g. catheter, endoscope, etc.) to be inserted and retracted, such that the flexible medical device is effectively steered in the direction of the lumen's distal section.

## Description

### TECHNICAL FIELD

The present invention relates to medical devices in the field of minimally invasive surgery, wherein an elongate flexible device, such as a catheter or endoscope, is inserted into the vasculature of the human body for surgical or diagnostic purposes. In particular, the invention relates to magnetically steerable devices for interventional cardiac procedures.

### PRIOR ART / BACKGROUND OF INVENTION

The field of magnetically-assisted minimally invasive surgery, wherein a magnetically responsive element disposed on a catheter, endoscope or other such device is steered inside a patient by generating external magnetic fields, is a well-known and increasingly popular area of medical development especially in the field of interventional cardiology.

One of the most common ailments of the heart is know as cardiac arrhythmia, a condition in which a portion of heart tissue contracts in a rapid and uncoordinated manner, out of sync with the rest of the heart. Many arrhythmias are now treated with transcatheter ablation, a minimally invasive procedure wherein a catheter is inserted through a small incision in the femoral artery and guided into the heart. Electrodes on the catheter tip identify the precise locations of anomalous electrical activity and RF energy is delivered through the electrodes to neutralize these areas, restoring the heart's natural rhythm.

Traditionally, a relatively stiff sheath having a curved distal end is first inserted into the patient's heart with the aid of an introducer. The introducer is then removed and the catheter is inserted into the sheath in its place. The position of the catheter is controlled by a combination of translating and rotating both the sheath and catheter, and changing the curvature of the catheter's distal section using one or more guide wires. Once the catheter tip is firmly in contact with the desired tissue site, an ablation is performed.

Recently a number of systems have been developed that use magnetic fields instead of guidewires to control ablation catheters in the heart. These magnetically steered catheters contain one or more distal magnetic elements that align with externally generated magnetic fields, allowing the surgeon to control the orientation of the catheter remotely. Remote magnetic navigation of ablation catheters can be more intuitive and efficient, and allows the surgeon to perform the procedure from another room, drastically reducing x-ray exposure time. Furthermore, magnetic steering enables the possibility of autonomous control of the catheter.

Magnetically steered ablation catheters have the advantages of being easier to control, able to reach otherwise difficult areas of the heart, and far less likely to induce a trauma such as perforation. However, one principal drawback of magnetically steered ablation catheters is their relatively low and unpredictable tissue contact force, especially when the target ablation site is located at a significant angle from the sheath's distal opening. The force applied at the target site by the catheter varies greatly with the catheter's angle of attack making ablations unpredictable and inconsistent. If the catheter approaches the site head-on, the forces are relatively large - on the order of 30-80g. But if the catheter must first turn 90 or more degrees to approach the target site, the contact forces rapidly decrease, becoming negligible as the attack angle approaches 180 degrees. This is because as the angle of attack increases, further inserting the catheter will only increase the working length and radius of curvature of the distal section of the catheter between the sheath's distal opening and the desired ablation site, not significantly increase the contact force of the catheter tip at the heart wall. Another important limitation of currently available magnetic ablation catheters is their relatively large bending radius. Although they are very flexible, the forces generated between the magnetically responsive elements in the catheter tip and the externally generated magnetic fields are relatively small, largely due to the limitations imposed by catheter size corresponding to a small magnetic volume.

A number of designs and configurations for magnetically steered medical instruments have been proposed in the literature, the most relevant of which are described here.

US 3,674,014 describes an elongate medical tube or sheath with a plurality of magnetically responsive elements separated by fluid-tight ball joints. The distal curvature of the sheath is controlled by external magnetic fields and the ball joints allow the device to bend without obstructing the inner passageway through which a medical instrument may be inserted.

US 6,385,472 describes a telescoping sheath for guiding a magnetic ablation catheter. The telescoping portion of the sheath has a magnetic element on its distal tip to aid in steering and orienting the catheter.

US 6,911,026 describes a catheter and sheath combination, both having distal magnetic elements, for atherectomy procedures.

US 2008/0006280A describes a simple embodiment of a sheath with a magnetic element at its distal tip for electrophysiology applications.

US 8,551,109 B2 is related to an apparatus and method for interventional navigation within a subject's body in which a medical device having at least one electrostrictive element is adapted to cause the distal end of the medical device to bend in a given direction for improving navigation. The medical device may further comprise at least one magnetically responsive element on the distal end, which may be oriented in the approximate direction of a magnetic field that is applied to the subject's body. A method for navigating a medical device though a subject's body is provided, by changing the direction of an applied magnetic field to align a magnetically responsive element on the distal end for orienting the distal end, and by applying a voltage to at least one electrostrictive element disposed in the distal portion of the device for causing the distal end to change orientation from that achieved by application of the magnetic field alone.

None of these devices adequately address the fundamental limitation of a magnetically-steered sheath: the sheath lumen must be flexible enough to allow sharp turns, but stiff enough to prevent kinking, allow the sheath to the pushed through the vasculature, and properly support the medical instrument inside. This limitation can be overcome by making the entire sheath relatively rigid, except for a short joint section - located just proximal to the magnetically responsive distal tip - which is extremely flexible and maintains its inner diameter. This allows the distal tip to make sharp turns of greater than 180 degrees without creating any additional friction for the catheter being inserted through the sheath.

### SUMMARY OF THE INVENTION

In light of the aforementioned prior art and the limitations thereof, it is the object of this invention to provide an improved device and method for performing minimally invasive interventions, especially cardiac ablations.

Around the middle of the 20^{th} century, an inventor named Joseph Friedman was granted a patent (US 2,094,268) for a drinking straw having an intermediate corrugated section that allowed the top portion of the straw to easily bend in any direction and stay put, an innovation that would forever revolutionize the beverage industry. Milkshakes could now be shared without bumping heads. Spitballs could be fired around corners. The world was a different place. Friedman and others followed his original patent with several more (US 2,550,797, US 3,025,004, US 3,409,224), progressively improving the design of the joint and describing simple methods for its mass-production.

Considered as a mechanical joint, the concertina-like corrugated section of a plastic straw has several subtle but interesting properties. It essentially acts as a spring with no restoring force. In order to deform the joint, whether bending or expanding/contracting, some force is required (analogous to a spring constant), but this force does not depend on the joint's initial state or orientation. Furthermore, when force is removed, the joint does not return to its initial state as a normal spring would - it simply maintains its current shape.

The properties of the corrugated bellows joint (as it will be called hereafter) relevant to the scope of this patent are briefly summarized here:
- Uniform flexibility - the joint's flexibility is the same in any direction, regardless of its current orientation.
- Expandability - The joint may be collapsed or expanded, changing its length and effective stiffness.
- Tight bending radius - The collapsible nature of the corrugated bellows allows the joint to make very sharp turns.
- Constant inner diameter - Because of the manner in which the corrugations either expand or fold into themselves, the joint maintains a constant inner diameter even at the limit of its bending radius.
- Stability/Stay-put action - The joint does not naturally return to any initial state. That is, once it is bent to an arbitrary angle or expanded to an arbitrary length, it will stay there until another force is applied.

The proposed device comprises an inner ablation catheter and an outer lumen that guides the ablation catheter to the target site. The inner ablation catheter comprises a very flexible distal section (100-150mm) and a relatively rigid proximal section and includes a standard ablation electrode array at its distal tip and all necessary electrical connections at its proximal end. The outer lumen or sheath comprises a short (10-20mm) rigid distal section with embedded magnetically responsive element(s), and a long, relatively rigid proximal section, connected by a specially designed joint. This joint must act as a very flexible tube having a very small bending radius and a constant inner diameter, i.e. the cross-sectional diameter of the joint must not change based on the joint's curvature. In a preferred embodiment, the joint is a bellows joint fundamentally similar to the corrugated plastic section forming the flexible part of a drinking straw, but other potential joint embodiments include a spring bonded to a flexible medical tube, or a flexible tube with a number of periodically spaced rigid rings.

The orientation of the magnetically responsive distal section of the outer lumen is controlled using externally generated magnetic fields, and when a desired orientation is obtained, the flexible inner ablation catheter can be easily inserted through the outer lumen and pushed against the tissue of the target site. The flexibility and tight turning radius of the outer lumen's distal joint allow the ablation catheter to reach sites near the sheath's entry point inside the heart which would otherwise be impossible or very difficult to access, and the joint's constant inner diameter does not inhibit the insertion of the ablation catheter, even at large angles.

The expandable nature of the corrugated bellows joint can also be exploited to increase the sheath's capabilities. Actuating the joint's length - by hydraulic, pneumatic or mechanical means - adds an important extra degree of freedom to the sheath.

By magnetically steering the sheath, instead of the catheter, the path of the catheter is constrained such that the catheter always approaches the target tissue site head-on, even if the target site is 180 degrees from the point of sheath insertion. With this navigation method, the tissue contact force no longer depends significantly on the location of the target site and the force resulting from a head-on approach is relatively easy to predict and control by selecting an appropriate catheter stiffness.

By selecting appropriately low-stiffness materials for the flexible joint and distal catheter section, it is possible to ensure that any forces on the interior of the heart wall are below an arbitrary value deemed acceptably low to mitigate the risk of perforation but also sufficient to be effective for ablation.

It is should also be noted that any tool of sufficient flexibility, not just ablation catheters, could be inserted into and controlled by the magnetically responsive outer lumen or sheath.

In all embodiments the elongate tube has a proximal end and a distal end and a lumen extending therebetween, the expandable, flexible joint provides a small bending radius and constant inner diameter connecting the proximal and distal sections of the tube and the at least one magnetically responsive element on the distal end of the tube provides the navigational properties of the device.

In a preferred embodiment the flexible joint comprises a corrugated bellows section whose corrugations permit the joint to expand and contract, bend freely in any direction and retain its cross-sectional shape in all orientations.

In another embodiment the flexible joint comprises a light spring bonded to the inside of a soft elastomeric tube. The spring can be provided with a flexible tubing or encased in two layers of polymer.

In another embodiment the flexible joint portion comprises a soft plastic tube with a plurality of embedded concentric rigid rings. The rings can also be completely encased in the elastomer tube material.

In another embodiment, the proximal portion of the tube comprises an inner lumen wall creating an annular lumen surrounding the inner lumen, wherein the annular lumen is closed at the proximal side as well as at the distal side beyond the flexible joint portion. Furthermore, the annular lumen comprises a connector at its proximal side usually connected to a pump unit provided to fill or evacuate the annular lumen with a fluid to lengthen or shorten the flexible joint portion. Then the material of the inner lumen wall is stretchable over its length to the maximum length of the device, when the flexible joint portion is lengthened to its maximum extent, in the same way as the flexible joint portion can be lengthened over its initial length to its maximum extent.

The one or more magnetically responsive element(s) are responsive to external magnetic fields to orient and stabilize the distal tip of the device. The joint connecting the proximal and distal ends of the tube has flexibility sufficient to permit the magnetically responsive distal element to align with any external magnetic field direction. Usually such external magnetic fields are on the order of 100mT.

The structure of the joint connecting the proximal and distal ends of the tube intrinsically resists any substantial change to its inner diameter as it bends so as not to impede the ability of a flexible medical device, such as an ablation catheter, to freely pass through the joint.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: is a perspective view of a preferred embodiment of the device, depicting the magnetically responsive distal tip aligning with an external magnetic field, thereby orienting the flexible instrument inside the sheath,
- Fig. 2a: is a schematic view of the embodiment of the device of Fig. 1 without a flexible medical instrument.
- Fig. 2b: is a schematic cross-sectional view of the device of Fig. 2a,
- Fig. 2c: is a schematic cross-sectional view of the device of Fig. 1, depicting the magnetically responsive distal tip aligning with an external magnetic field,
- Fig. 3: is a schematic cross-sectional view of a second embodiment of the device, wherein the flexible distal joint comprises a spring embedded in or surrounded by a flexible elastomeric material,
- Fig. 4: is a schematic cross-sectional view of a third embodiment of the device, wherein the flexible distal joint comprises a plurality of concentric rigid rings embedded in or surrounded by a flexible elastomeric material,
- Fig. 5a: is a schematic cross-sectional view of a fourth embodiment of the device having a second interior lumen,
- Fig. 5b: is an illustration of the changing length of the distal joint of the device of Fig. 5a as fluid is pumped into the space between the inner and outer lumens,
- Fig. 5c: is a schematic cross-sectional view of the device of Fig. 5a, depicting the magnetically responsive distal tip aligning with an external magnetic field,
- Fig. 6: is a schematic view of a fifth embodiment of the device having two or more flexible distal joints and magnetically responsive elements,
- Fig. 7a: is a schematic cross-sectional view of a sixth embodiment of the device having a rigid telescoping distal section controlled proximally by means of a control wire,
- Fig. 7b: depicts the telescoping section of the device according to Fig. 7a in the fully extended position,
- Fig. 7c: depicts the telescoping section of the device according to Fig. 7a in the fully retracted position.
- Fig. 7d: is a schematic view of the device according to Fig. 7a, depicting the magnetically responsive distal tip aligning with an external magnetic field and the rigid telescoping section fully extended,
- Fig. 8: is a schematic cross-sectional view of a seventh embodiment of the device wherein the corrugated bellows joint of the device of Fig. 1 is enclosed in a flexible sleeve bonded to the each end of the joint,
- Figs. 9a to 9d: are illustrations of the device's ability to navigate into the right ventricle to treat arrhythmias in the RVOT in four different stages.

### DESCRIPTION OF PREFERRED EMBODIMENTS

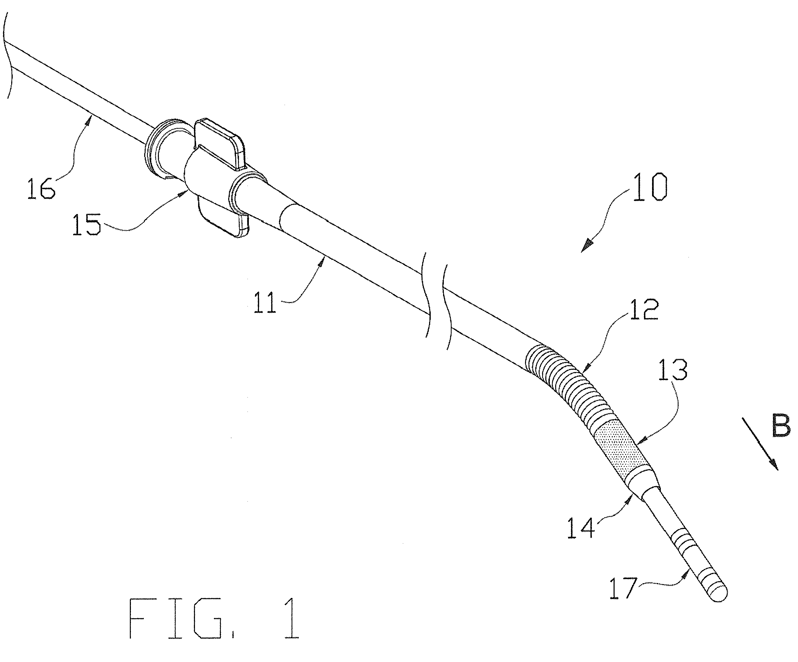

Fig. 1 shows a perspective view of a preferred sheath embodiment 10, comprising a proximal tubular sheath 11 connected to a corrugated bellows joint 12 and a short distal section containing one or more magnetically responsive elements 13. A soft atraumatic tip 14 is disposed at the distal tip of the device and is preferably radiopaque for fluoroscopy localization. A standard Luer fitting 15 is disposed at the proximal end of the sheath 11 to facilitate easy attachment to hemostatic valves. A flexible instrument having a proximal section 16 and a functional distal tip 17 is inserted through the sheath 10 and the distal tip 17 is thus steered by the magnetically responsive element(s) 13.

The magnetically responsive distal tip 13 is aligning with an external magnetic field of direction according to arrow B, thereby orienting the flexible instrument inside the sheath. Tip 14 comprises a tapered section made from a soft elastomeric material to minimize potential trauma as the device is inserted into the patient's vasculature and is preferably radiopaque for fluoroscopy localization. Such radiopaque atraumatic tips are common in traditional introducer sheaths and well known to those familiar with the art. The magnetically responsive element 13 comprises a hollow permanent magnet.

The three or more sections of the device described in the first embodiment, and all further embodiments, are composed of diverse materials and must be permanently bonded together without obstructing the inner lumen of the device. Bonding of these elements may be achieved by thermal bonding, cyanoacrylate adhesives or UV-curing adhesives, among other methods, all of which will be well known to those familiar with the art.

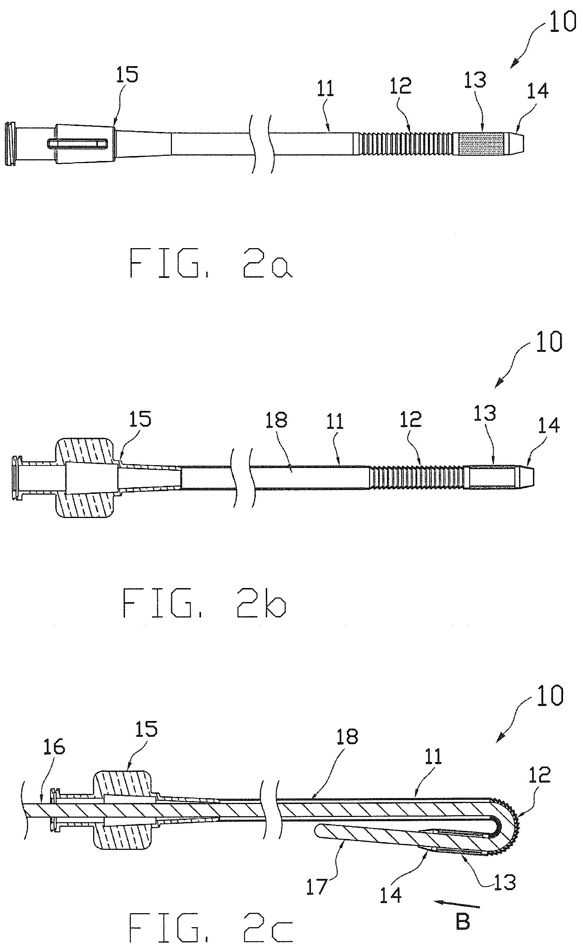

Fig. 2a is a schematic view of sheath 10 of Fig. 1 without the flexible instrument of Fig. 1. Fig. 2b is a cross-sectional view of sheath 10 of Fig. 2a wherein the nature of the corrugated joint 12 and the inner lumen 18 are clearly depicted.

The proximal section 11 of the sheath is a stiff plastic tube much like traditional introducer sheaths, providing the sheath shell. A small distal section 13, 14 of the sheath is separated from the proximal section 11 with the highly flexible joint 12 with a predetermined minimum inner diameter and small bending radius. The magnetically responsive element 13 is embedded in the distal sheath section. The inner diameter of the proximal section 11 as well as of the distal section 13, 14 is preferably of the same size as the minimum inner diameter of the flexible joint 12. The magnetic element 13 is shown as a hollow permanent magnet attached between the corrugated bellows joint 12 and the atraumatic distal tip 14.

Fig. 2c is a cross-sectional view of sheath 10 being steered by an external magnetic field with the direction of arrow B, wherein the magnetic element 13 is oriented in the direction determined by the magnetic field. In this view it is clear how the corrugations in joint 12 allow it to make sharp turns greater than 180 degrees while maintaining its minimum inner diameter so that the flexible instrument 16 can be freely inserted and retracted.

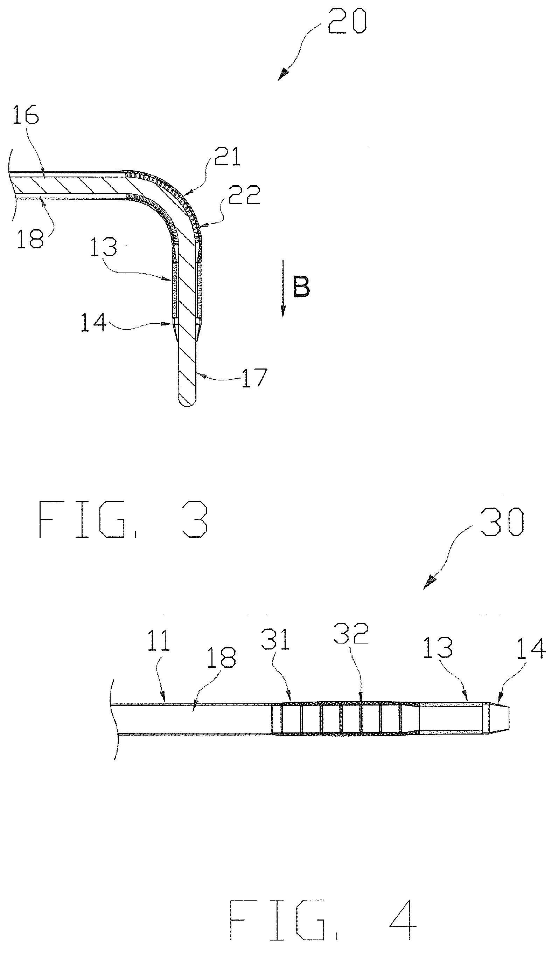

Fig. 3 is a cross-sectional view of the distal portion of a second embodiment of the device 20, identical in every way to 10 except that the corrugated bellows joint 12 is replaced with a spring 22 bonded to or enclosed in an extremely flexible elastomer 21. The spring 22 is flexible enough to allow the magnetically responsive element 13 to align with an externally generated magnetic field of direction according to arrow B, and the coils of the spring 22 prevent the elastomer 21 from kinking, allowing a flexible medical instrument 16 to be freely inserted through the device 20.

According to Fig. 3 the material of the proximal sheath is formed integrally with the flexible elastomer part 21, which is in turn directly attached to the hollow sleeve-like magnetic element 13.

In the embodiment shown in Fig. 3, the spring 22 is simply bonded to the inside of the elastomer 21, thus defining a minimum free lumen. It is also possible that the spring is embedded in a flexible plastic tube or that a further layer of the flexible elastomer is attached at the inside of the spring and attached to the proximal and distal sheath parts. For instance, if the spring is dip-coated with a flexible elastomer, a process well known to those familiar with the art, the resulting elastomer 21 will fully enclose the spring, forming a smooth surface on both the outside of the flexible joint and the inside lumen.

Fig. 4 is a cross-sectional view of the distal portion of a third embodiment of the device 30, identical in every way to 20 except that the spring is replaced with a series of concentric rigid rings 32 bonded to or enclosed in an extremely flexible elastomer 31. The rigidity of the concentric rings prevents the elastomer 31 from kinking and obstructing the passage a flexible medical instrument 16, and the flexibility of the elastomer is sufficient to allow the magnetically responsive element 13 to align with an externally generated magnetic field B.

The concentric rigid rings of Fig. 4 can be made of metal, preferably non-ferromagnetic metal, or hard plastic and partly or fully embedded in or surrounded by the flexible elastomeric material 31. Within the embodiment shown, the cross-section of each of the rings 32 is a circle and these rings 32 have about 2/3 of their cross-section embedded in the elastomer 31. As in the embodiment of Fig. 3, the elastomer 31 is directly attached to the proximal sheath and the distal magnetic sleeve 13.

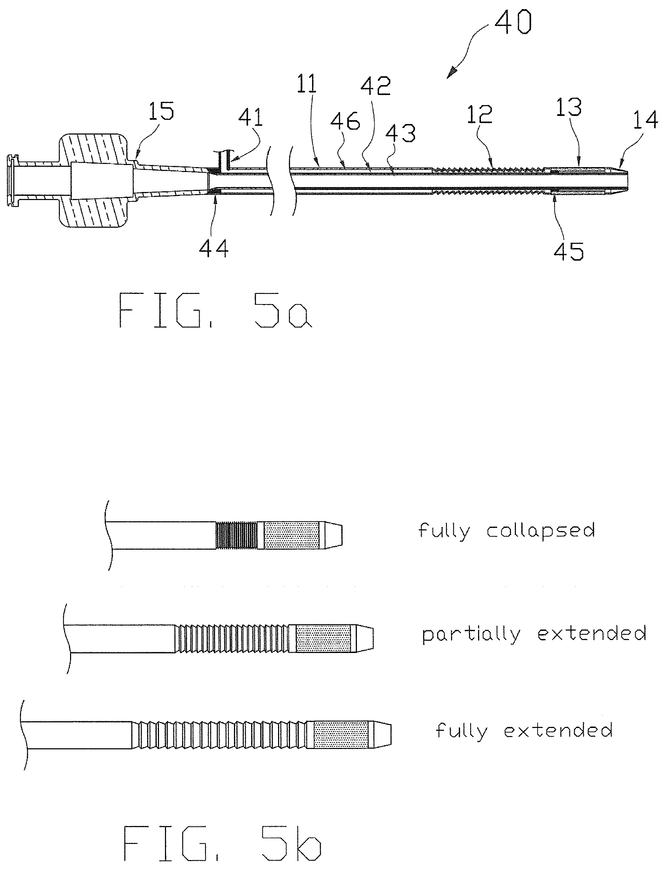

Fig. 5a is a cross-sectional view of a fourth embodiment of the device 40 having an additional inner tube 42 disposed from the proximal to distal end of the device. Inner tube 42 is attached and sealed to outer tube 11 at or near the luer fitting 15 at the proximal end 44 and at or near the magnetic sleeve 13 at the distal end 45, thus forming an intermediate, sealed lumen 46 surrounding the inner tube for introduction of the instrument/catheter. Gas or liquid is pumped in through valve 41 and the length of joint 12 varies with the pressure in intermediate lumen 46. A flexible medical device can be freely inserted through inner lumen 43. Joint 12 is the corrugated bellows joint of the device of Fig. 1 with the proviso that the bellows joint is attached to the outer sheath sleeve and the outer portion of the magnetic element 13.

In the embodiment of Fig. 5a, the inner wall, separating the intermediate fluid extension lumen 46 from the instrument lumen is extended beyond the joint 12 towards the tip 14. Then the hollow magnetic element 13 is attached via the distal seal 45 on one side and via the ring of the atraumatic tip 14 on the other side at said inner wall. In other words, the magnetic element 13 does not directly form the wall with the instrument lumen as in the other embodiments. Instead, a flexible inner liner runs throughout the length of the device and forms a smooth and continuous inner surface. Such an inner liner is of course also possible within the embodiments of Figs. 1 to 4.

Fig. 5b illustrates three states of joint 12: fully collapsed - corresponding to a negative relative pressure in intermediate lumen 46, partially extended - corresponding to ambient pressure in 46, and fully extended - corresponding to positive relative pressure in 46 and maximum length of joint 12. Of course, the length of joint 12 can transition continuously between fully collapsed and fully extended states.

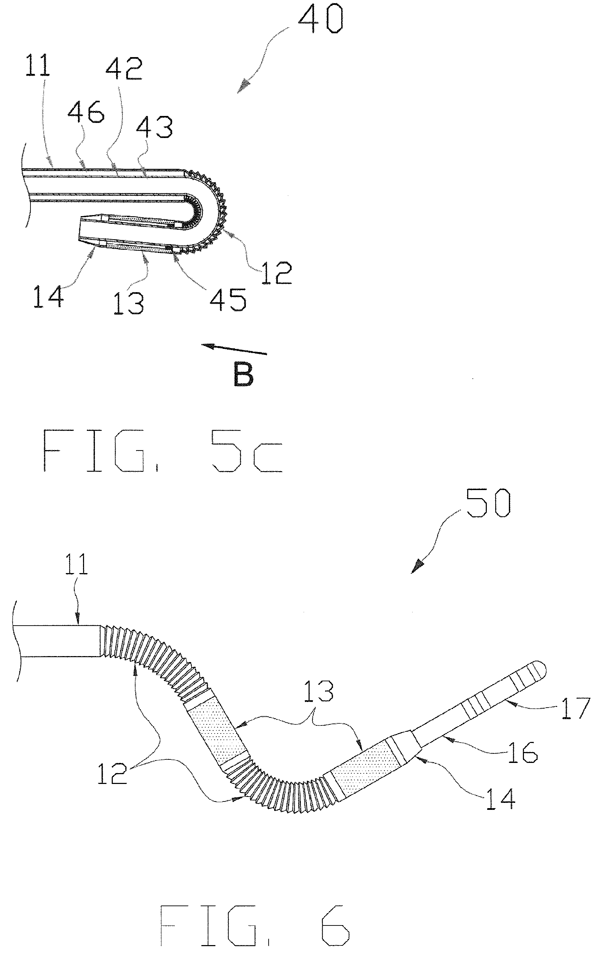

Fig. 5c is a cross-sectional view of the distal portion of the fourth sheath embodiment 40 of Fig. 5a aligning with an externally generated magnetic field of a direction according to arrow B and making a sharp turn greater than 180 degrees.

Fig. 6 is a schematic view of a further embodiment 50 of the device wherein two or more magnetically responsive elements 13 and/or two or more flexible distal joints 12 are disposed at the distal end of the device. The joints 12 are shown as corrugated bellows joints according to Fig. 1, but it is also possible to replace them by any of the other joint embodiments. It is also possible to provide the lumen extension embodiment for e.g. the first proximal joint 12 and an embedded spring or ring-based joint for the more distal joint of the embodiment of Fig, 6.

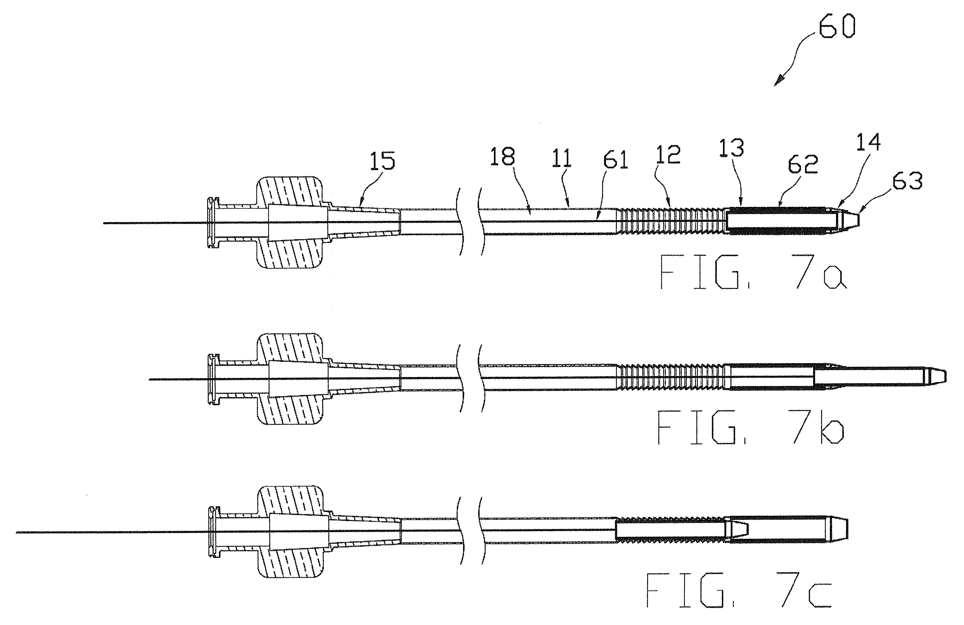

Fig. 7a is a cross-sectional view of a sixth embodiment of the device 60 having a rigid tubular telescoping section 62 located in the lumen 18 near the distal tip of the device. The telescoping section can be extended or retracted by means of a control wire 61 which runs through the length of the device and out the luer fitting 15 to allow proximal control of the telescoping section 62. The telescoping section 62 has an atraumatic tip 63 similar to tip 14.

Fig. 7b depicts the telescoping section 62 in the fully extended position. This increases the useful range of the device by supporting the catheter at distances from the distal tip 14 which would otherwise be too great for the flexible medical instrument to apply significant forces to tissue walls.

Fig. 7c depicts the telescoping section 62 in the fully retracted position. In this position the rigid section 62 extends throughout the length of the flexible joint 12, making this section effectively rigid. This configuration is especially useful when the device is first being inserted into the patient's vasculature and it is desirable to have a uniform rigidity throughout the device.

The embodiment of Fig. 7a to 7c shows a length of the rigid telescopic section which is essentially equal to the length of the magnetic element 13 or distal portion (including distal tip 14) and which is also essentially equal to the length of the flexible joint, to be able to stiffen the entire flexible joint. Of course, the rigid telescopic section can then also be longer than the flexible joint portion. The wire 61 is preferably guided in the wall of instrument lumen proximal to flexible joint 12.

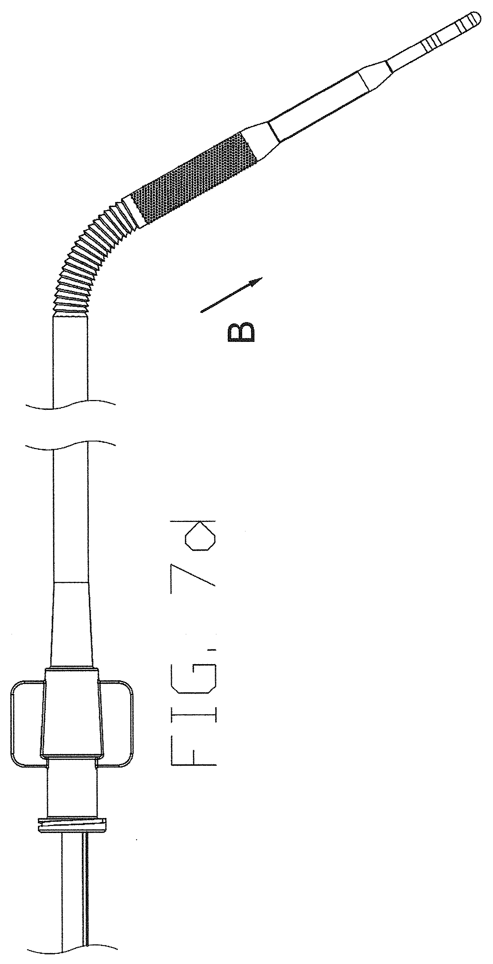

Fig. 7d is a schematic view of embodiment 60 illustrating the distal magnet 13 aligning with an external magnetic field and the rigid telescoping section 62 fully extended to support the catheter and substantially increase the effective range of the device.

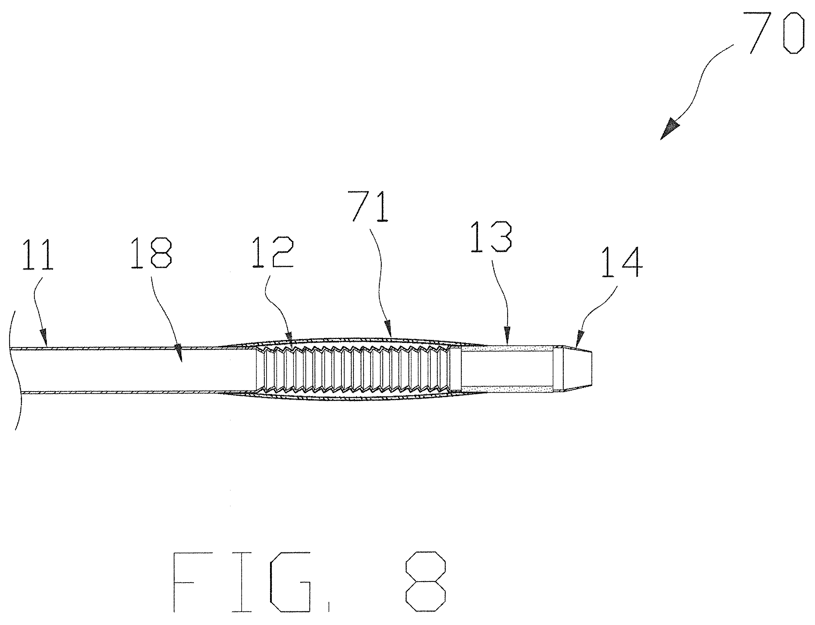

Fig. 8 is a schematic cross-sectional view of the distal portion of a seventh embodiment of the device 70 wherein the corrugated bellows joint 12 is enclosed in a smooth flexible sleeve 71. The sleeve 71 is bonded to each end of the joint but the intermediate joint section is not bonded to the sleeve, allowing the joint to flex without being compromised by the sleeve. The sleeve 71 thus forms a smooth and continuous outer surface when the joint 12 is straight, which is desirable to minimize trauma when the device is being inserted into or withdrawn from a patient's vasculature. The camber of sleeve 71 in Fig. 8 is exaggerated to emphasize the fact that the sleeve is only bonded to the ends of joint 12 and is not intended to illustrate the preferred embodiment. It is preferred that the diameter of the bellows joint part 12 has essentially the same outer diameter as the magnetic element 13 and the proximal sheath tube 11. The sleeve 71 can be stretched over the corrugated part leaving almost no space between sleeve 71 and the corrugated bellows joint 12 material.

Sleeve 71 is preferably formed from a microporous or fibrous material, such as ePTFE, which has mechanical properties more similar to cloth than to a traditional plastic tube, and as such will not significantly increase the stiffness of the joint. Materials such as ePTFE are also well suited for this purpose due to their low friction and biocompatibility. In addition, the use of an ePTFE material for sleeve 71 would allow a gas exchange with the surrounding space when the bellows joint 12 bends, and the sleeve is bent as well. Of course such an outer sleeve could also be implemented in any of the other embodiments, wherein the flexible joint comprises a spring or a plurality of rings instead of a corrugated bellows. The sleeve 71 may also be extended beyond the magnetic element(s) 13 all the way to the atraumatic tip 14, in order to cover the magnet(s) and prevent direct contact with the patient's vasculature.

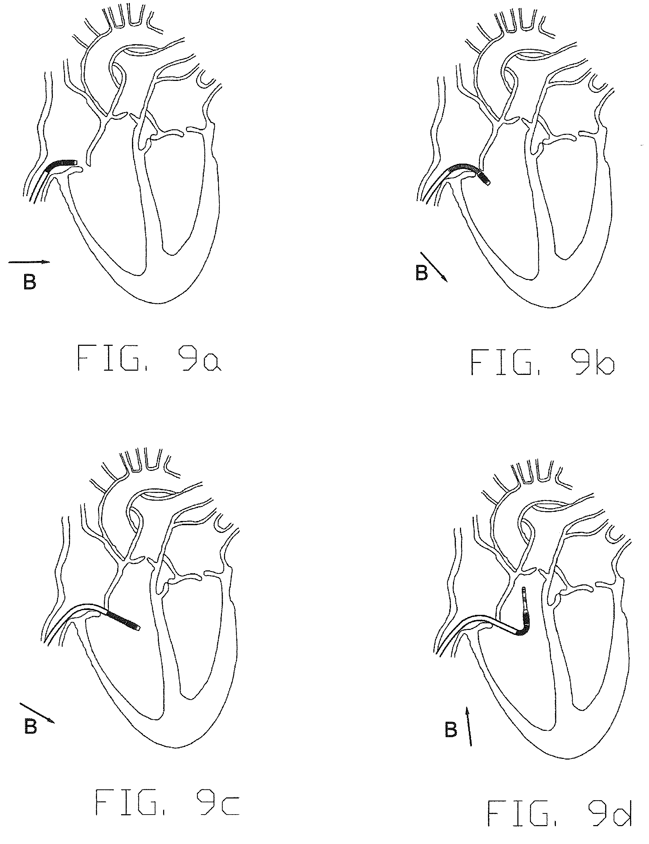

Figs. 9a-d illustrate the ability of embodiment 5 to navigate multiple turns inside the heart by utilizing a combination of magnetic steering and expansion of the distal joint. The right ventricular outflow tract (RVOT) is an area of the heart that is currently difficult to reach using magnetically guided catheters. In Fig. 9a, the distal tip of the device is aimed at the valve leading to the right ventricle. In Fig. 9b, the bellows joint is expanded, pushing the distal tip into the right ventricle. In Fig. 9c, the whole device is inserted further into the vasculature until the flexible joint is entirely in the right ventricle. At this point the distal tip may be aimed at the RVOT and the catheter may be inserted to perform an ablation, as illustrated in Fig. 9d.

**LIST OF REFERENCE SIGNS**

| | | | |
|---|---|---|---|
| 10 | first embodiment | 40 | fourth embodiment |
| 11 | proximal sheath tube | 41 | valve |
| 12 | corrugated bellows joint | 42 | inner tube |
| 13 | magnetic element | 43 | inner lumen |
| 14 | atraumatic distal tip | 44 | proximal seal |
| 15 | luer fitting | 45 | distal seal |
| 16 | flexible ablation catheter | 46 | intermediate lumen |
| 17 | ablation electrode array | 50 | fifth embodiment |
| 18 | lumen | 60 | sixth embodiment |
| 20 | second embodiment | 61 | control wire |
| 21 | flexible elastomer | 62 | rigid telescoping section |
| 22 | embedded spring | 63 | atraumatic tip |
| 30 | third embodiment | 70 | seventh embodiment |
| 31 | flexible elastomer | 71 | flexible sleeve |
| 32 | embedded ring | | |

## Claims

1. A device for positioning cannular medical devices, such as catheters and endoscopes within body lumens or cavities, comprising at least two segments (11; 13, 14) to be navigated through the body lumens or cavities, one or more magnetically responsive element(s) (13) in the distal end portion of the device, especially to cause the distal end portion of the device to bend in a given direction for navigation, **characterized in that**, the segments (11; 13, 14) are hollow tubes having an inner diameter connecting a proximal portion (11) to the front opening of a distal portion (14) allowing a medical device introduced into the inner cavity of the sheath to extend beyond the front opening and wherein the proximal portion (11) is connected with the distal portion (13, 14) through at least one flexible joint portion (12, 22, 32).

2. The system according to claim 1, wherein the flexible joint portion (12, 22, 32) comprises a corrugated bellows joint (12).

3. The device according to claim 1, wherein the flexible joint portion (12, 22, 32) comprises a flexible tubing (21) attached to the proximal portion (11) as well as to the distal portion and wherein a spring (22) is provided within the flexible tubing (21).

4. The device according to claim 1, wherein the flexible joint portion (12, 22, 32) comprises a soft plastic tube (31) with a plurality of embedded concentric rigid rings (32).

5. The device according to any one of claims 1 to 4, wherein more than one flexible joint (12, 22, 32) is disposed between the proximal and distal ends of the tube.

6. The device according to claim 5, wherein each flexible joint (12, 22, 32) has at least one magnetically responsive element (13) disposed at its distal end.

7. The device according to any one of claims 1 to 6, wherein the length of the joint connecting the proximal and distal ends of the tube (11) is controllable with pneumatic actuation, puller wires, or any other means.

8. The device according to any one of claims 1 to 7, wherein the distal portion of the tube contains a telescoping element (62) slidably mounted inside the front opening of the distal portion (14) and actuated with an actuation element (61) that is pushed or pulled from the proximal section, especially a guide wire or pneumatic actuator.

9. The device according to any one of claims 1 to 7, wherein the proximal portion of the tube (11) contains an inner lumen wall (42) creating an annular lumen (43) surrounding the inner lumen (18), wherein the annular lumen (43) is closed at the proximal side (44) as well as at the distal side (45) beyond the flexible joint portion (12, 22, 32), wherein the annular lumen (43) comprises a connector (41) at its proximal side adapted to fill or evacuate the annular lumen (43) with a fluid to lengthen or shorten the flexible joint portion (12, 22, 32).

10. The device according to claim 9, wherein the material of the inner lumen wall (42) is stretchable over its length to the maximum length of the device, when the flexible joint portion (12, 22, 32) is lengthened to its maximum extent, in the same way as the flexible joint portion (12, 22, 32) can be lengthened over its initial length to its maximum extent.
